(19) Europäisches Patentamt European Patent Office Office européen des brevets

(11) **EP 3 533 384 B1**

(12) **EUROPEAN PATENT SPECIFICATION**

(45) Date of publication and mention
of the grant of the patent:
**08.09.2021 Bulletin 2021/36**

(21) Application number: **17866127.8**

(22) Date of filing: **26.10.2017**

(51) Int Cl.:
*A61B 3/10* (2006.01)     *A61B 1/00* (2006.01)
*A61F 9/008* (2006.01)     *G01N 21/17* (2006.01)

(86) International application number:
**PCT/JP2017/038637**

(87) International publication number:
**WO 2018/079639 (03.05.2018 Gazette 2018/18)**

(54) **MEDICAL INFORMATION PROCESSING DEVICE AND MEDICAL INFORMATION PROCESSING PROGRAM**

VORRICHTUNG ZUR VERARBEITUNG MEDIZINISCHER INFORMATIONEN UND VERFAHREN ZUR VERARBEITUNG MEDIZINISCHER INFORMATIONEN

DISPOSITIF ET PROGRAMME DE TRAITEMENT D'INFORMATION MÉDICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2016 JP 2016210286**

(43) Date of publication of application:
**04.09.2019 Bulletin 2019/36**

(73) Proprietors:
• **Nidek Co., Ltd.**
**Gamagori-shi, Aichi 443-0038 (JP)**
• **University of Tsukuba**
**Tsukuba-shi, Ibaraki 305-8577 (JP)**

(72) Inventors:
• **YASUNO, Yoshiaki**
**Tsukuba-shi**
**Ibaraki 305-8577 (JP)**
• **MAKITA, Shuichi**
**Tsukuba-shi**
**Ibaraki 305-8577 (JP)**

(74) Representative: **Viering, Jentschura & Partner mbB**
**Patent- und Rechtsanwälte**
**Am Brauhaus 8**
**01099 Dresden (DE)**

(56) References cited:
**JP-A- 2016 083 510     JP-A- 2016 114 557**

• **HEIKE H. MÜLLER ET AL: "Imaging thermal expansion and retinal tissue changes during photocoagulation by high speed OCT", BIOMEDICAL OPTICS EXPRESS, vol. 3, no. 5, 1 May 2012 (2012-05-01), page 1025, XP055692336, United States ISSN: 2156-7085, DOI: 10.1364/BOE.3.001025**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present disclosure relates to a medical information processing device and a medical information processing program for measuring a change in a biological tissue occurring as a result of applying energy.

Background Art

Conventionally, various research has been carried out for measuring a change in a biological tissue occurring from the application of energy (such as treatment light, for example). For example, in research disclosed in Non-Patent Literature 1, a change in an ocular fundus tissue caused by heat in photocoagulation treatment of the ocular fundus is measured using Doppler OCT.

Citation List

Non-Patent Literature

Non-Patent Literature 1: "Imaging thermal expansion and retinal tissue changes during photocoagulation by high speed OCT," 1 May 2012, Vol. 3, No. 5, BIOMEDICAL OPTICS EXPRESS

Summary of Invention

In the method disclosed in Non-Patent Literature 1, only a final change of the biological tissue is measured. Thus, for example, when the biological tissue has expanded and then contracts to the same state before the expansion, in the method disclosed in Non-Patent Literature 1, it is difficult to appropriately measure a transition of the change in the biological tissue.

A typical object of the present disclosure is to provide a medical information processing device and a medical information processing program that are capable of appropriately measuring a transition of a change in biological tissue.

A medical information processing device provided by a first aspect of typical embodiments of the present disclosure is a medical information processing device capable of measuring a change in a biological tissue occurring when energy is applied, the medical information processing device including a processor, wherein the processor is configured to acquire a plurality of OCT signals, at different times, detected by reflected light of measurement light irradiated toward a measurement section of the biological tissue, calculate a rate of change over time of a local optical path length of the measurement light by processing the plurality of OCT signals, and calculate a cumulative deformation amount based on the rate of change over time of the local optical path length, the cumulative deformation amount being obtained through accumulation in a time direction of a deformation amount ($\geq 0$) of the biological tissue in the measurement section.

A medical information processing program provided by a second aspect of typical embodiments of the present disclosure is a medical information processing program executed in a medical information processing device capable of measuring a change in a biological tissue occurring when energy is applied, the medical information processing program, when executed by a processor of the medical information processing device, causing the medical information processing device to perform an OCT signal acquisition step of acquiring a plurality of OCT signals at different times, the OCT signals being detected using reflected light of measurement light irradiated toward a measurement section of the biological tissue, a local optical path length rate of change calculation step of calculating a rate of change over time of a local optical path length of the measurement light, by processing the plurality of OCT signals, and a cumulative deformation amount calculation step of calculating a cumulative deformation amount based on the rate of change over time of the local optical path length, a cumulative deformation amount being obtained through accumulation in a time direction of a deformation amount ($\geq 0$) of the biological tissue in the measurement section.

According to the medical information processing device and the medical information processing program according to the present disclosure, a transition in a change of the biological tissue can be appropriately measured.

A processor of an ophthalmological information processing device exemplified in the present disclosure is configured to acquire a plurality of OCT signals at different times in relation to the same measurement section of a biological tissue. The OCT signal is a signal detected by reflected light of measurement light irradiated toward the measurement section. The processor is configured to calculate a rate of change over time of a local optical path length (hereinafter also simply referred to as a "local optical path length rate of change") of the measurement light, by processing the plurality of OCT signals. The local optical path length is an optical path length of the measurement light that is incident on and reflected by each of local parts (namely, of the measurement light that passes through each of the local parts) that have respectively different positions in a depth direction (a direction along an optical axis of the measurement light, hereinafter also referred to as a "Z direction"). The processor is configured to calculate a cumulative deformation amount, which is obtained through accumulation in a time direction of a value ($\geq 0$) indicating a deformation amount of the biological tissue in the measurement section, on the basis of the rate of change over time of the local optical path length. As a result, a transition in a change of the biological tissue can be appropriately ascertained by the cumulative deformation amount of the biological tissue.

Various devices may function as the medical information processing device. For example, a medical device including an OCT unit configured to acquire the OCT signal, a treatment unit configured to irradiate treatment light onto a living organism (such as a laser treatment unit), and a control unit, may function as the medical information processing device. Further, an OCT device configured to acquire the OCT signal, and a treatment device configured to irradiate the treatment light onto the living organism may be separate devices. In this case, at least one of the OCT device or the treatment device may function as the medical information processing device, or a device (a personal computer or the like, for example) that is separate from the OCT device and the treatment device may function as the medical information processing device. Control units of a plurality of devices may execute processing to measure the change in the biological tissue in concert with each other.

The processor may be configured to calculate the local optical path length rate of change by performing spatial differentiation of values obtained by time differentiation of the plurality of OCT signals. In this case, the local optical path length rate of change can be appropriately calculated using the Doppler OCT principle.

The processor may be configured to perform smoothing processing (smoothing) of the values obtained by the time differentiation of the plurality of OCT signals, and perform the spatial differentiation of the values on which the smoothing processing has been performed. In this case, noise when performing the spatial differentiation can be reduced.

A specific method of the smoothing processing may be selected as appropriate. For example, the processor may be configured to perform the smoothing processing by performing averaging at each of locations in the direction along the optical axis of the measurement light (hereinafter also referred to as the "Z direction"). Further, when multiplying a complex conjugate with the plurality of OCT signals in the time differentiation, the processor may be configured to perform the smoothing processing by dividing a spectrum and lowering resolution.

The processor may be configured to calculate a deformation amount of the biological tissue at each of times, by adding up absolute values of the local optical path length rate of change in the Z direction. In this case, the cumulative deformation amount of the biological tissue can be appropriately calculated, regardless of a direction (one of a positive Z direction or a negative Z direction) of the deformation occurring in the biological tissue.

Values indicating the deformation amount of the biological tissue at each of the times may be calculated, without using the absolute values of the local optical path length rate of change. For example, values indicating the deformation amount of the biological tissue at each of the times may be calculated by adding up squares of the local optical path length rate of change in the Z direction.

The processor may be configured to calculate a correction value of the deformation amount on the basis of the deformation amount of the biological tissue in a period before a time point at which the energy is started to be applied, and calculate the cumulative deformation amount using the calculated correction value. For example, the processor may be configured to calculate the cumulative deformation amount by accumulating, in a time direction, the deformation amounts corrected using the correction value. In this case, the cumulative deformation amount is calculated in a state in which an influence of noise of the deformation amount in the period before the application of the energy is reduced. Thus, a more accurate cumulative deformation amount can be calculated.

The energy causing the change in the biological tissue may be the treatment light irradiated onto the biological tissue. The processor may be configured to control the irradiation of the treatment light based on the calculated cumulative deformation amount. In this case, the irradiation of the treatment light can be appropriately controlled on the basis of the transition in the change of the biological tissue.

Brief Description of Drawings

Fig. 1 is a diagram showing an overall configuration of a medical device 1.
Fig. 2 is a flowchart showing an example of cumulative deformation amount calculation processing.
Fig. 3 is a diagram showing an example of schematized information obtained by time differentiation of a plurality of OCT signals and smoothing processing.
Fig. 4 is a diagram showing an example of a schematized result obtained by adding up absolute values of a local optical path length rate of change in a Z direction.
Fig. 5 is a diagram showing a result of correction performed on the information illustrated in Fig. 4.
Fig. 6 is an example of a graph of a calculated cumulative deformation amount.

Modes for Carrying Out Invention

Hereinafter, an exemplary embodiment of the present disclosure will be described with reference to the drawings. For example, a medical device 1 of the present embodiment measures a change in an ocular fundus when treatment light is irradiated onto the ocular fundus of a patient's eye E. However, at least a part of technology exemplified in the present embodiment may be applied when measuring a change in a tissue of the patient's eye E other than the ocular fundus. Further, at least part of the technology exemplified in the present embodiment may be applied when measuring a change in a biological tissue other than the patient's eye E (skin, a digestive organ, the brain, or the like, for example). In other words, fields to which the technology exemplified in the present embodiment can be applied are not limited to the ophthalmological field.

Further, in the present embodiment, the medical device 1 that includes an OCT unit 10, a laser treatment unit 20, and a control unit 30 functions as a medical information processing device that measures a change in a biological tissue. However, another device may function as the medical information processing device. For example, an OCT device that includes an OCT unit, and a treatment device that irradiates treatment light onto a living organism may be separate devices. In this case, a control unit of at least one of the OCT device or the treatment device may perform processing to measure the change in the biological tissue. A device (a personal computer or the like, for example) connected to the OCT device may function as the medical information processing device.

Overall configuration

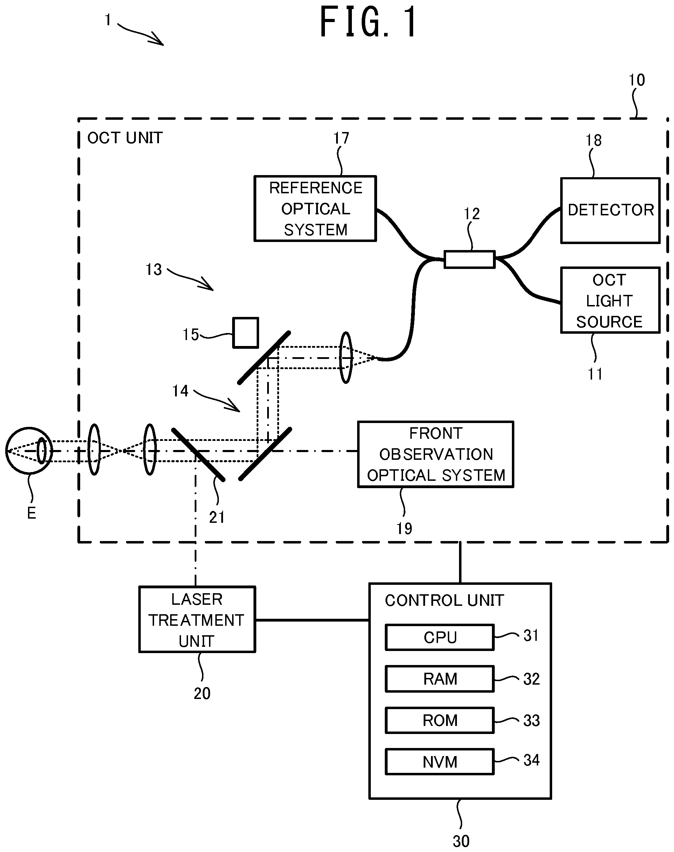

An overall configuration of the medical device 1 of the present embodiment will be explained with reference to Fig. 1. As described above, the medical device 1 of the present embodiment includes the OCT unit 10, the laser treatment unit 20, and the control unit 30.

The OCT unit 10 will be described. The OCT unit 10 uses the principle of optical coherence tomography (OCT) to acquire an OCT signal. The OCT unit 10 includes an OCT light source 11, a coupler (light splitter) 12, a measurement optical system 13, a reference optical system 17, a detector 18, and a front observation optical system 19.

The OCT light source 11 emits light (OCT light) to acquire the OCT signal. The coupler 12 splits the light emitted from the OCT light source 11 into a measurement light and a reference light. Further, the coupler 12 of the present embodiment combines the measurement light reflected by a biological tissue (the ocular fundus of the patient's eye E in the present embodiment) and the reference light generated by the reference optical system 17, and causes the combined light to be received by the detector 18.

The measurement optical system 13 guides the measurement light split by the coupler 12 to the biological tissue, and returns the measurement light reflected by the biological tissue to the coupler 12. The measurement optical system 13 includes an optical scanner 14. The optical scanner 14 can cause the measurement light to be deflected as a result of being driven by a drive unit15. In the present embodiment, two galvanometer mirrors, which can deflect the measurement light in mutually different directions, are used as the optical scanner 14. However, another device that deflects the light (at least one of a polygon mirror, a resonant scanner, an acousto-optic device, or the like, for example) may be used as the optical scanner 14.

In the present embodiment, of the biological tissue, at least part of a section onto which the treatment light is irradiated is assumed to be a measurement section. The control unit 30 drives the drive unit 15 to continuously irradiate the measurement light onto the same measurement section (performs a so-called "motion (M-) scan"). As a result, a plurality of OCT signals are intermittently acquired from the same measurement section at a constant time interval.

The reference optical system 17 generates the reference light and returns the reference light to the coupler 12. The reference optical system 17 of the present embodiment generates the reference light by causing the reference light divided by the coupler 12 to be reflected by a reflection optical system (a reference mirror, for example). However, the configuration of the reference optical system 17 may be changed. For example, the reference optical system 17 may cause the light incident from the coupler 12 to pass through without being reflected and return the light to the coupler 12. The reference optical system 17 can change the optical path length difference between the measurement light and the reference light by moving an optical member in an optical path. In the present embodiment, the optical path length difference is changed by moving the reference mirror in the optical axis direction. The configuration for changing the optical path length difference may be provided in an optical path of the measurement optical system 13.

The detector (photodetector) 18 detects an interference signal of the measurement light and the reference light. In the present embodiment, the Fourier domain OCT principle is adopted. In Fourier domain OCT, a spectral intensity of the interference light (a spectral interference signal) is detected by the detector 18 and a complex OCT signal is acquired using a Fourier transform on data of the spectral intensity. Spectraldomain-OCT (SD-OCT), swept-source-OCT (SS-OCT), and the like may be used as examples of the Fourier domain OCT. For example, time-domain-OCT (TD-OCT) and the like may also be adopted. In the present embodiment, SD-OCT is adopted. In the case of SD-OCT, for example, a low coherent light source (broadband light source) is used as the OCT light source 11. Further, in the case

4

of SD-OCT, a spectro-optical system (a spectrometer), which divides the interference light into frequency components (wavelength components), is provided in the vicinity of the detector 18 on the optical path of the interference light. In the case of SS-OCT, for example, a wavelength scanning light source (wavelength tunable light source) that changes an emitted wavelength at high speed temporally is used as the OCT light source 11. In this case, the OCT light source 11 may include a light source, a fiber ring resonator, and a wavelength selecting filter. For example, the wavelength selecting filter may be a filter using a diffraction grating and a polygon mirror in combination, a filter using a Fabry-Perot etalon structure, and the like.

The front observation optical system 19 is provided to acquire a front image of the biological tissue (the ocular fundus of the patient's eye E in the present embodiment). For the configuration of the front observation optical system 19, for example, a configuration of at least one of a scanning laser ophthalmoscope (SLO), a fundus camera, or the like may be adopted.

The laser treatment unit 20 will be explained. The laser treatment unit 20 emits the treatment light (treatment laser light in the present embodiment). For example, in the present embodiment, the treatment light emitted from the laser treatment unit 20 is used to perform at least one of photocoagulation treatment of the ocular fundus of the patient's eye E, or subthreshold photocoagulation treatment. In the subthreshold photocoagulation treatment, the treatment light is irradiated using a level of energy that does not coagulate the ocular fundus tissue. In the present embodiment, a dichroic mirror 21 is provided on an optical path of the measurement light in the OCT unit 10. The treatment light emitted by the laser treatment unit 20 is reflected by the dichroic mirror 21, and is irradiated onto the biological tissue. The medical device 1 can acquire the OCT signal using the OCT unit 10, while irradiating the treatment light onto the biological tissue using the laser treatment unit 20. A configuration required to perform the irradiation of the treatment light and the acquisition of the OCT signal in parallel may be selected as appropriate. For example, an optical system used to irradiate the OCT measurement light onto the biological tissue, and an optical system used to irradiate the treatment light onto the biological tissue may be each independently provided. Further, the energy used to change the biological tissue is not limited to the treatment light. For example, mechanical energy may be applied from outside, using a piezoelectric element or the like. The energy may be applied using ultrasonic waves, electromagnetic waves, wind pressure, or the like.

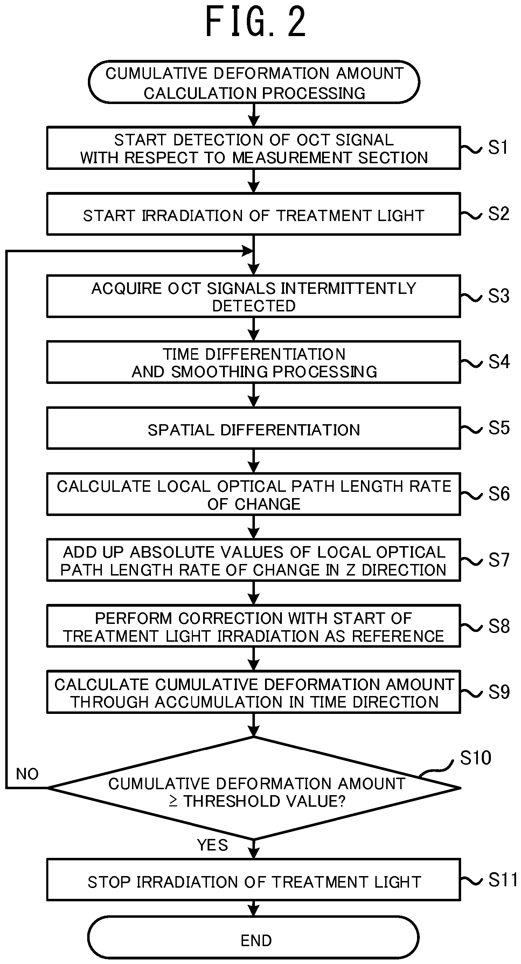

The control unit 30 will be explained. The control unit 30 performs various controls of the medical device 1. The control unit 30 include a CPU 31, a RAM 32, a ROM 33, and a non-volatile memory (NVM) 34. The CPU 31 is a controller that performs various controls. The RAM 32 temporarily stores various information. The ROM 33 stores programs executed by the CPU 31, various default values, and the like. The NVM 34 is a non-transitory storage medium that can hold storage content even if a power supply is cut off. The NVM 34 may store a medical information processing program that is used to execute cumulative deformation amount calculation processing (refer to Fig. 2) to be described below. As described above, a control unit that performs the cumulative deformation amount calculation processing need not necessarily be provided in the medical device 1. For example, at least one of control units of a PC, an OCT device, a laser treatment device, or the like may perform the cumulative deformation amount calculation processing.

Cumulative deformation amount calculation processing

The cumulative deformation amount calculation processing will be explained with reference to Fig. 2 to Fig. 6. In the cumulative deformation amount calculation processing, a cumulative deformation amount is calculated, which is obtained through accumulation, in a time direction, of a deformation amount ($\geq 0$) of the biological tissue in the measurement section. Further, in the cumulative deformation amount calculation processing of the present embodiment, the irradiation of the treatment light is controlled on the basis of the calculated cumulative deformation amount. The CPU 31 of the control unit 30 performs the cumulative deformation amount calculation processing shown in Fig. 2, in accordance with the medical information processing program stored in the NVM 34.

First, the CPU 31 starts detection of the OCT signal with respect to the biological tissue (the ocular fundus of the patient's eye E in the present embodiment) (S1). More specifically, the CPU 31 sets a part of the biological tissue to be irradiated with the treatment light as the measurement section, and irradiates the OCT measurement light onto the measurement section. Using the detector 18, the CPU 31 detects the interference signal of the reflected light of the measurement light reflected from the measurement section, and the reference light generated by the reference light optical system 17. By performing the Fourier transform on the interference signal, the CPU 31 detects the complex OCT signal. The above processing is intermittently performed (the M-scan) with respect to the same measurement section. The control unit that controls the detection of the OCT signal using the OCT unit 10 and the control unit that calculates the cumulative deformation amount may be different control units.

Next, the CPU 31 starts the irradiation of the treatment light with respect to the biological tissue (S2). In the present embodiment, the CPU 31 performs the irradiation of the treatment light onto a region of the biological tissue including the measurement section by controlling the operation of the laser treatment unit 20.

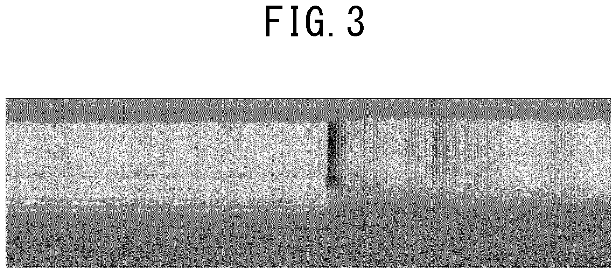

The CPU 31 acquires the plurality of OCT signals detected intermittently (S3). The CPU 31 performs time differentiation of the plurality of OCT signals, and smoothing processing (S4). More specifically, as shown by (Expression 1), the CPU 31 of the present embodiment multiplies an OCT signal Γ by a value obtained by displacing the OCT signal Γ by Δn lines and taking its complex conjugate. Further, in order to perform averaging at each of locations in the direction (hereinafter referred to as a "Z direction") along the optical axis of the measurement light, the CPU 31 multiplies a local average filter (width: 2s + 1 pixel). In this way, the smoothing processing is performed. Here, δt is the length of a time period over which the measurement light is irradiated when detecting the single OCT signal (a scanning time period per single A line). Phase information and intensity information are both included in the equation shown in (Expression 1). Fig. 3 is an example of schematized information obtained using (Expression 1). The vertical axis in Fig. 3 shows the Z direction and the horizonal axis shows time.

[Expression 1]

$$H_t\left(z_m, t_n; \Delta n \delta t\right) \equiv \sum_{j=-s}^{s} \Gamma^*\left(z_{m+j}, t_{n-\Delta n/2}\right) \Gamma\left(z_{m+j}, t_{n+\Delta n/2}\right)$$

Next, the CPU 31 performs spatial differentiation of the values obtained by the time differentiation (S5). More specifically, as shown by (Expression 2), the CPU 31 of the present embodiment multiplies (Expression 1) by a value obtained by displacing (Expression 1) by Δm pixels in a depth direction and taking its complex conjugate, and extracts the phase thereof. Here, $δ_z$ is the depth (the length in the Z direction) per single pixel.

[Expression 2]

$$\Delta_z \Delta_t \phi\left(z_m, t_n; \Delta m \delta z, \Delta n \delta t\right) \equiv \arg\left[H^*\left(z_{m-\Delta m/2}, t_n\right) H\left(z_{m+\Delta m/2}, t_n\right)\right]$$

Next, the CPU 31 calculates a rate of change over time of a local optical path length (lOPL) (hereinafter referred to as a "local optical path length rate of change") (S6). The local optical path length is an optical path length of the OCT measurement light that is incident on and reflected by each of local parts that have respectively different positions in the depth direction (the Z direction). The local optical path length rate of change is calculated using (Expression 3) below, from a phase Φ. Here, $λ_0$ is a central wavelength (1020 nm in the present embodiment) of the OCT light source 11, and n is a refractive index of the tissue. Note that $δz_0 = nδz$ is a distance in air per single pixel (3.2 um/pix in the present embodiment).

[Expression 3]

$$r\left(t_i, z_j\right) = \frac{\lambda_0}{4n\pi} \frac{\Delta_t \Delta_z \phi\left(t_i, z_j\right)}{\Delta m \delta z \cdot \Delta n \delta t} \approx \frac{1}{lOPL(t,z)} \frac{\partial lOPL(t,z)}{\partial t}\left[s^{-1}\right]$$

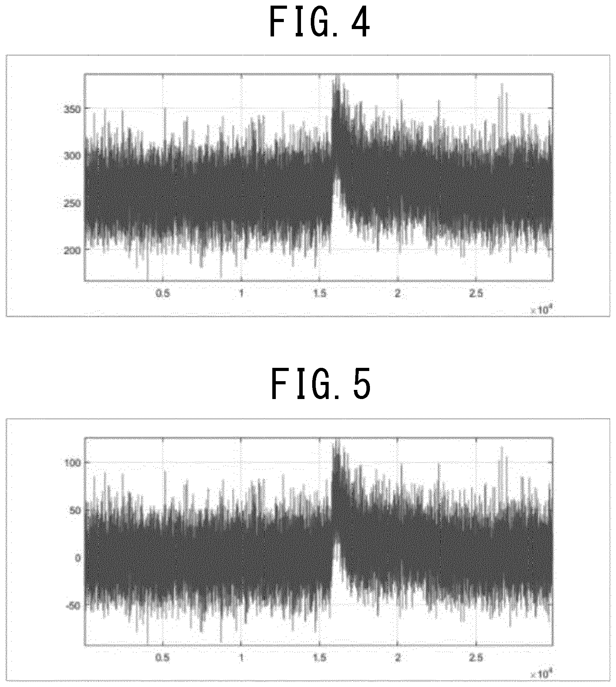

Next, as shown by (Expression 4), the CPU 31 adds up absolute values of the local optical path length rate of change in the Z direction (S7). The value shown in (Expression 4) indicates the deformation amount (≥ 0) of the biological tissue at each of time points. Fig. 4 is an example of schematized information obtained using (Expression 4). The vertical axis in Fig. 4 shows $S^{-1}$, and the horizontal axis shows time.

[Expression 4]

$$R(t) = \sum_z \left|r(z,t)\right|$$

Next, the CPU 31 performs correction, taking as a reference a time point at which an application of energy is started (that is, a time point at which the irradiation of the treatment light is started in the present embodiment) (S8). More specifically, the CPU 31 calculates a correction value of the deformation amount of the biological tissue on the basis of the deformation amount of the biological tissue in a period before a time point to at which the irradiation of the treatment light is started, as shown in (Expression 5), and corrects the deformation amount of the biological tissue at each of the time points, using the calculated correction value. As a result of this, an influence of noise of the deformation amount in the period before the irradiation of the treatment light is reduced. Fig. 5 is a diagram showing a schematized result of the correction performed on the information illustrated in Fig. 4.

[Expression 5]

$$R'(t) = R(t) - \overline{R(t)}\big|_{t<t_0}$$

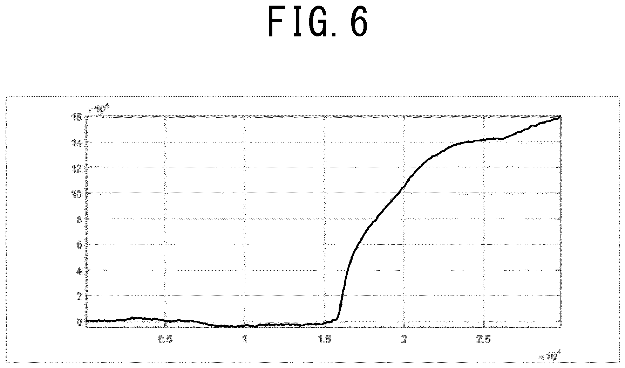

Next, as shown in (Expression 6), by accumulating the values corrected at S8 in the time direction, the CPU 31 calculates a cumulative deformation amount $M(t_i)$ (S9). Fig. 6 is a graph of the calculated cumulative deformation amount, where the vertical axis shows the cumulative deformation amount and the horizontal axis shows time. A transition in a change of the biological tissue is reflected in the calculated cumulative deformation amount. Further, in the present embodiment, correction is performed (S8) that takes as the reference the time point at which the irradiation of the treatment light is started. As a result, taking the time point at which the irradiation of the treatment light is started as zero, the cumulative deformation amount sequentially increases after the start of the irradiation.

[Expression 6]

$$M(t_i) = \sum_{k=0}^{i} R'(t_k)$$

Next, the CPU 31 controls the irradiation of the treatment light on the basis of the calculated cumulative deformation amount (S10, S11). For example, in the present embodiment, the CPU 31 determines whether the cumulative deformation amount is equal to or greater than a threshold value (S10). The threshold value may be set as appropriate in accordance with a treatment objective (for example, which of the photocoagulation treatment and subthreshold photocoagulation treatment is to be performed) and the like. If the cumulative deformation amount is lower than the threshold value (no at S10), the processing returns to S3, and the processing is repeated to calculate the cumulative deformation amount (S3 to S9) while the irradiation of the treatment light is continued. When the cumulative deformation amount becomes equal to or greater than the threshold value (yes at S10), the CPU 31 stops the irradiation of the treatment light (S11), and ends the processing. A specific method of controlling the irradiation of the treatment light on the basis of the cumulative deformation amount may be changed as appropriate. For example, on the basis of the cumulative deformation amount, the CPU 31 may adjust, as appropriate, an intensity of the treatment light to be irradiated.

As described above, the medical device 1 of the present embodiment (the medical information processing device) does not cause the measurement section of the biological tissue to be scanned intermittently by the OCT measurement light, but continuously irradiates the measurement light onto the same measurement section (performs the M-scan in the present embodiment), and intermittently obtains the plurality of OCT signals. Thus, the transition in the change of the biological tissue while the treatment light is being irradiated is monitored in real time. As a result, for example, it becomes easy to perform the control of the irradiation of the treatment light on the basis of the measured transition in the change, and the like.

For example, in the treatment of the biological tissue using the treatment light, even when the treatment light of a constant energy is being irradiated, it is known that there is a case in which the biological tissue switches between expansion and contraction. Thus, when only a final change in the biological tissue is measured, there may be a case in which it is difficult to appropriately ascertain a treatment result. In contrast to this, the cumulative deformation amount calculated by the medical device 1 of the present embodiment is a value corresponding to an energy amount that causes the change in the biological tissue. Thus, according to the present embodiment, the transition in the change of the biological tissue can be appropriately ascertained.

The technology disclosed in the above-described embodiment is merely an example. Thus, the technology exemplified in the above-described embodiment may be changed. For example, specific methods for each type of numerical processing (the time differentiation at S4, the spatial differentiation at S5, and the like) may be changed as appropriate.

In the above-described embodiment, the deformation amount ($\geq 0$) of the biological tissue at each of the times is calculated by adding up the absolute values of the local optical path length rate of change in the Z direction (S7). However, the processing at S7 may be changed. For example, the CPU 31 may add up squares of the local optical path length rate of change in the Z direction. In this case also, a value corresponding to the energy amount causing the changes in the biological tissue can be appropriately calculated.

**Claims**

1. A medical information processing device (1) capable of measuring a change in a biological tissue occurring when energy is applied, the medical information processing device (1) comprising:

   a processor (31),
   wherein
   the processor (31) is configured to:

   acquire a plurality of OCT signals, at different times, detected by reflected light of measurement light irradiated toward a measurement section of the biological tissue;
   calculate a rate of change over time of a local optical path length of the measurement light by processing the plurality of OCT signals; **characterized in that** the processor is configured to
   calculate a cumulative deformation amount based on the rate of change over time of the local optical path length, the cumulative deformation amount being obtained through accumulation in a time direction of a deformation amount of the biological tissue in the measurement section.

2. The medical information processing device (1) according to claim 1, wherein
   the processor (31) is configured to calculate the rate of change over time of the local optical path length by performing spatial differentiation of values obtained by time differentiation of the plurality of OCT signals.

3. The medical information processing device (1) according to claim 2, wherein
   the processor (31) is configured to perform the spatial differentiation after performing smoothing processing of the values obtained by the time differentiation of the plurality of OCT signals.

4. The medical information processing device (1) according to any one of claims 1 to 3, wherein
   the processor (31) is configured to calculate the deformation amount of the biological tissue at each of times, by adding up absolute values of the rate of change over time of the local optical path length in a direction along an optical axis of the measurement light.

5. The medical information processing device (1) according to any one of claims 1 to 4, wherein
   the processor (31) is configured to calculate a correction value of the deformation amount of the biological tissue based on the deformation amount of the biological tissue in a period before a time point at which the energy is started to be applied, and calculate the cumulative deformation amount using the correction value.

6. The medical information processing device (1) according to any one of claims 1 to 5, wherein

   the energy causing a change to occur in the biological tissue is treatment light irradiated onto the biological tissue, and
   the processor (31) is further configured to control irradiation of the treatment light based on the calculated cumulative deformation amount.

7. A medical information processing program executed in a medical information processing device (1) capable of measuring a change in a biological tissue occurring when energy is applied, the medical information processing program, when executed by a processor (31) of the medical information processing device (1), causing the medical information processing device (1) to perform:

   an OCT signal acquisition step of acquiring a plurality of OCT signals at different times, the OCT signals being detected using reflected light of measurement light irradiated toward a measurement section of the biological tissue;
   a local optical path length rate of change calculation step of calculating a rate of change over time of a local optical path length of the measurement light, by processing the plurality of OCT signals; **characterized in that** medical information processing program causes the medical information processing device to perform
   a cumulative deformation amount calculation step of calculating a cumulative deformation amount based on the rate of change over time of the local optical path length, a cumulative deformation amount being obtained through accumulation in a time direction of a deformation amount of the biological tissue in the measurement section.

**Patentansprüche**

1. Medizinische Informationsverarbeitungsvorrichtung (1), die in der Lage ist, eine Änderung in einem biologischen Gewebe zu messen, die auftritt, wenn Energie zugeführt wird, wobei die medizinische Informationsverarbeitungsvorrichtung (1) aufweist:

   einen Prozessor (31),
   wobei
   der Prozessor (31) konfiguriert ist, um:

   zu verschiedenen Zeiten eine Mehrzahl von OCT-Signalen zu erlangen, die durch reflektiertes Licht von Messlicht, das in Richtung zu einem Messabschnitt des biologischen Gewebes gestrahlt wird, detektiert werden,
   eine Änderungsrate über die Zeit einer lokaler-optischer-Pfad-Länge des Messlichts durch Verarbeiten der Mehrzahl von OCT-Signalen zu berechnen,
   **dadurch gekennzeichnet, dass** der Prozessor konfiguriert ist, um
   einen Gesamtdeformationsbetrag basierend auf der Änderungsrate über die Zeit der lokaler-optischer-Pfad-Länge zu berechnen, wobei der Gesamtdeformationsbetrag durch Akkumulieren in einer Zeitrichtung eines Deformationsbetrags des biologischen Gewebes in dem Messabschnitt erhalten wird.

2. Medizinische Informationsverarbeitungsvorrichtung (1) gemäß Anspruch 1, wobei
   der Prozessor (31) konfiguriert ist, um die Änderungsrate über die Zeit der lokaler-optischer-Pfad-Länge zu berechnen, durch Durchführen einer räumlichen Differenzierung von Werten, die durch eine Zeitdifferenzierung der Mehrzahl von OCT-Signalen erhalten werden.

3. Medizinische Informationsverarbeitungsvorrichtung (1) gemäß Anspruch 2, wobei
   der Prozessor (31) konfiguriert ist, um die räumliche Differenzierung nach dem Durchführen einer Glättungsverarbeitung der durch die Zeitdifferenzierung der Mehrzahl von OCT-Signalen erhaltenen Werte durchzuführen.

4. Medizinische Informationsverarbeitungsvorrichtung (1) gemäß irgendeinem der Ansprüche 1 bis 3, wobei
   der Prozessor (31) konfiguriert ist, um den Deformationsbetrag des biologischen Gewebes zu jedem von Zeitpunkten zu berechnen, durch Addieren von absoluten Werte der Änderungsrate über die Zeit der lokaler-optischer-Pfad-Länge in einer Richtung entlang einer optischen Achse des Messlichts.

5. Medizinische Informationsverarbeitungsvorrichtung (1) gemäß irgendeinem der Ansprüche 1 bis 4, wobei
   der Prozessor (31) konfiguriert ist, um einen Korrekturwert des Deformationsbetrags des biologischen Gewebes basierend auf dem Deformationsbetrag des biologischen Gewebes in einem Zeitraum vor einem Zeitpunkt, zu dem mit der Energiezufuhr begonnen wird, zu berechnen, und den Gesamtdeformationsbetrag unter Verwendung des Korrekturwerts zu berechnen.

6. Medizinische Informationsverarbeitungsvorrichtung (1) gemäß irgendeinem der Ansprüche 1 bis 5, wobei

   die Energie, die das Auftreten einer Veränderung in dem biologischen Gewebe verursacht, Behandlungslicht ist, das auf das biologische Gewebe gestrahlt wird, und
   der Prozessor (31) ferner konfiguriert ist, um das Strahlen des Behandlungslichts basierend auf dem berechneten Gesamtdeformationsbetrag zu steuern.

7. Medizinisches Informationsverarbeitungsprogramm, das in einer medizinischen Informationsverarbeitungsvorrichtung (1) ausgeführt wird, die in der Lage ist, eine Änderung in einem biologischen Gewebe zu messen, die auftritt, wenn Energie zugeführt wird, wobei das medizinische Informationsverarbeitungsprogramm, wenn es von einem Prozessor (31) der medizinischen Informationsverarbeitungsvorrichtung (1) ausgeführt wird, bewirkt, dass die medizinische Informationsverarbeitungsvorrichtung (1) Folgendes durchführt:

   einen OCT-Signalerlangungsschritt des Erlangens einer Mehrzahl von OCT-Signalen zu verschiedenen Zeiten, wobei die OCT-Signale unter Verwendung von reflektiertem Licht von Messlicht, das in Richtung zu einem Messabschnitt des biologischen Gewebes gestrahlt wird, detektiert werden,

einen lokaler-optischer-Pfad-Länge-Änderungsrate-Berechnungsschritt zum Berechnen einer Änderungsrate über die Zeit einer lokaler-optischer-Pfad-Länge des Messlichts, durch Verarbeiten der Mehrzahl von OCT-Signalen,

**dadurch gekennzeichnet, dass** das medizinische Informationsverarbeitungsprogramm bewirkt, dass die medizinische Informationsverarbeitungsvorrichtung Folgendes durchführt:

einen Gesamtdeformationsbetrag-Berechnungsschritt zum Berechnen eines Gesamtdeformationsbetrags basierend auf der Änderungsrate über die Zeit der lokaler-optischer-Pfad-Länge, wobei ein Gesamtdeformationsbetrag durch Akkumulieren in einer Zeitrichtung eines Deformationsbetrags des biologischen Gewebes in dem Messabschnitt erhalten wird.

## Revendications

1. Dispositif de traitement d'informations médicales (1) capable de mesurer un changement dans un tissu biologique se produisant lorsque de l'énergie est appliquée, le dispositif de traitement d'informations médicales (1) comprenant :

   un processeur (31),
   dans lequel
   le processeur (31) est configuré pour :

   acquérir une pluralité de signaux OCT, à différents moments, détectés par la lumière réfléchie de la lumière de mesure irradiée vers une section de mesure du tissu biologique ;
   calculer un taux de changement dans le temps d'une longueur de chemin optique local de la lumière de mesure en traitant la pluralité de signaux OCT ;
   **caractérisé en ce que** le processeur est configuré pour
   calculer une quantité de déformation cumulée sur la base du taux de changement dans le temps de la longueur de chemin optique local, la quantité de déformation cumulée étant obtenue par accumulation dans une direction temporelle d'une quantité de déformation du tissu biologique dans la section de mesure.

2. Dispositif de traitement d'informations médicales (1) selon la revendication 1, dans lequel le processeur (31) est configuré pour calculer le taux de changement dans le temps de la longueur de chemin optique local en effectuant une différenciation spatiale de valeurs obtenues par différenciation temporelle de la pluralité de signaux OCT.

3. Dispositif de traitement d'informations médicales (1) selon la revendication 2, dans lequel le processeur (31) est configuré pour effectuer la différenciation spatiale après avoir effectué un traitement de lissage des valeurs obtenues par la différenciation temporelle de la pluralité de signaux OCT.

4. Dispositif (1) de traitement d'informations médicales selon l'une quelconque des revendications 1 à 3, dans lequel le processeur (31) est configuré pour calculer la quantité de déformation du tissu biologique à chacun parmi des instants, en additionnant des valeurs absolues du taux de changement dans le temps de la longueur de chemin optique local dans une direction le long d'un axe optique de la lumière de mesure.

5. Dispositif de traitement d'informations médicales (1) selon l'une quelconque des revendications 1 à 4, dans lequel. le processeur (31) est configuré pour calculer une valeur de correction de la quantité de déformation du tissu biologique sur la base de la quantité de déformation du tissu biologique dans une période précédant un point temporel auquel l'énergie commence à être appliquée, et calculer la quantité de déformation cumulée en utilisant la valeur de correction.

6. Dispositif de traitement d'informations médicales (1) selon l'une quelconque des revendications 1 à 5, dans lequel

   l'énergie provoquant un changement dans le tissu biologique est une lumière de traitement irradiée sur le tissu biologique, et
   le processeur (31) est en outre configuré pour commander l'irradiation de la lumière de traitement sur la base de la quantité de déformation cumulée calculée.

**7.** Programme de traitement d'informations médicales exécuté dans un dispositif de traitement d'informations médicales (1) capable de mesurer un changement dans un tissu biologique se produisant lorsque de l'énergie est appliquée, le programme de traitement d'informations médicales, lorsqu'il est exécuté par un processeur (31) du dispositif de traitement d'informations médicales (1), amenant le dispositif de traitement d'informations médicales (1) à effectuer :

une étape d'acquisition de signal OCT consistant à acquérir une pluralité de signaux OCT à différents moments, les signaux OCT étant détectés en utilisant la lumière réfléchie de la lumière de mesure irradiée vers une section de mesure du tissu biologique ;

une étape de calcul de taux de changement de longueur de chemin optique local consistant à calculer un taux de changement dans le temps d'une longueur de chemin optique local de la lumière de mesure, en traitant la pluralité de signaux OCT ;

**caractérisé en ce que** le programme de traitement d'informations médicales amène le dispositif de traitement d'informations médicales à exécuter

une étape de calcul de quantité de déformation cumulée consistant à calculer une quantité de déformation cumulée sur la base du taux de changement dans le temps de la longueur de chemin optique local, une quantité de déformation cumulée étant obtenue par accumulation dans une direction temporelle d'une quantité de déformation du tissu biologique dans la section de mesure.

# FIG. 1

# FIG. 2

CUMULATIVE DEFORMATION AMOUNT
CALCULATION PROCESSING

START DETECTION OF OCT SIGNAL
WITH RESPECT TO MEASUREMENT SECTION ～ S1

START IRRADIATION OF TREATMENT LIGHT ～ S2

ACQUIRE OCT SIGNALS INTERMITTENTLY
DETECTED ～ S3

TIME DIFFERENTIATION
AND SMOOTHING PROCESSING ～ S4

SPATIAL DIFFERENTIATION ～ S5

CALCULATE LOCAL OPTICAL PATH LENGTH RATE
OF CHANGE ～ S6

ADD UP ABSOLUTE VALUES OF LOCAL OPTICAL
PATH LENGTH RATE OF CHANGE IN Z DIRECTION ～ S7

PERFORM CORRECTION WITH START OF
TREATMENT LIGHT IRRADIATION AS REFERENCE ～ S8

CALCULATE CUMULATIVE DEFORMATION AMOUNT
THROUGH ACCUMULATION IN TIME DIRECTION ～ S9

S10

CUMULATIVE DEFORMATION AMOUNT
≥ THRESHOLD VALUE?

NO

YES

STOP IRRADIATION OF TREATMENT LIGHT ～ S11

END

# FIG. 3

FIG. 4

FIG. 5

# FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Imaging thermal expansion and retinal tissue changes during photocoagulation by high speed OCT. *BIOMEDICAL OPTICS EXPRESS,* 01 May 2012, vol. 3 (5